# EUROPEAN PATENT APPLICATION

(11) **EP 4 834 199 A1**
(43) Date of publication of application: **04.11.2026**
(21) Application number: 26174394.2
(22) Date of filing: 24.04.2026
(51) Int. Cl.: A61B 3/00

(54) **HEAD SUPPORT FOR AN OPHTHALMIC IMAGING INSTRUMENT**

(30) Priority: 28.04.2025 GB 202506339
(71) Applicant: Optos PLC, Dunfermline, Scotland KY11 8GR (GB)
(72) Inventor: Boland, Estelle, Dunfermline, KY11 8GR (GB); Anderson, Alan, Dunfermline, Scotland, KY11 8GR (GB); Gourlay, Iain, Dunfermline (GB); Muyo, Gonzalo, Dunfermline, KY11 8GR (GB); Suttie, Alan, Glasgow, G4 9SS (GB); Shearlaw, Morven, Glasgow, G4 9SS (GB); Slorach, Louis, Glasgow, G4 9SS (GB); Loudon, Brian, Glasgow, G4 9SS (GB)
(74) Representative: Hoffmann Eitle

(57) **Abstract**

An ophthalmic imaging instrument for imaging an eye of a subject, comprising a patient interface with which a face of a head of the subject is to engage during the imaging of the eye, the patient interface being arranged to fix an orientation of the head relative to the ophthalmic imaging instrument by contacting a first portion of the face overlying a frontal bone of the head and a respective second portion of the face overlying each zygomatic bone of the head when the face of the subject engages with the patient interface. The ophthalmic imaging instrument further comprises an image acquisition apparatus arranged to acquire an image of the eye while the face of the subject is engaged with the patient interface.

## Description

### [Field]

Example aspects herein generally relate to the field of ophthalmic imaging instruments and, in particular, to patient interfaces of such instruments, with which the head of a subject engages during imaging of the subject's eye.

### [Background]

Ophthalmic imaging devices employ various imaging techniques to image different parts of the eye, and are used by clinicians to diagnose and manage a variety of eye conditions. Ophthalmic imaging devices include (but are not limited to) scanning laser ophthalmoscopes (SLOs), optical coherence tomography (OCT) imaging devices, fundus cameras, microperimetry devices, and corneal topography devices (among others), or a combination of two or more such devices.

Ophthalmic imaging devices typically include a patient interface (also referred to as a facial interface or head support), such as a chinrest, a headrest, or an eyecup, for example, which has a contact surface that the patient's head contacts during imaging of the patient's eye. Patient interfaces allow the location of the eye to be set to approximately the correct position with respect to the ophthalmic imaging device, and help keep the subject's head steady during the imaging, thereby reducing artefacts in the acquired images caused by movements of the patient's head relative to the device. The provision of a patient interface which effectively keeps the subject's head steady is particularly important for ophthalmic imaging devices such as microperimeters and optical coherence tomography (OCT) imaging devices, for example, that require relatively long imaging sessions which can last from tens of seconds to several minutes (depending on the device), where movements of the eye during imaging may result in motion artifacts in the acquired ocular image.

### [Summary]

There is provided, in accordance with a first example aspect herein, an ophthalmic imaging instrument for imaging an eye of a subject, comprising a patient interface with which a face of a head of the subject is to engage during the imaging of the eye, the patient interface being arranged to fix an orientation of the head relative to the ophthalmic imaging instrument by contacting a first portion of the face overlying a frontal bone of the head and respective left and right portions of the face overlying each zygomatic bone of the head when the face of the subject engages with the patient interface. The ophthalmic imaging instrument further comprises an image acquisition apparatus arranged to acquire an image of the eye while the face of the subject is engaged with the patient interface.

In some example embodiments, the patient interface is arranged to avoid contacting a nose of the subject and a third portion of the face overlying a maxilla of the head when the face of the subject is engaged with the patient interface. The patient interface may also be arranged to avoid contacting a chin of the subject. For example, the patient interface may not comprise a chin rest such that when the subject engages the patient interface the subject's chin is not contacted by the patient interface. In these example embodiments, the patient interface may have an elongate (and preferably continuous) contact surface comprising a central portion arranged to contact the first portion of the face when the face of the subject is engaged with the patient interface, the contact surface extending continuously (preferably only downwardly) to each of a first end portion of the contact surface and a second end portion of the contact surface, the first end portion and the second end portion being arranged to respectively contact the left and right portions of the face when the face of the subject is engaged with the patient interface. In this case, the end portions of the contact surface may be immoveable (or fixed) with respect to the central portion of the contact surface, the patient interface being rigid. The patient interface may alternatively be configured to distort, for example flex and/or plastically indent, when engaged by the face of the subject, with the end portions of the contact surface being moved with respect to the central portion of the contact surface by the subject's face; in this case, the shape of the contact surface may be changeable only by pressure applied to parts of the contact surface.

The elongate contact surface may further comprise a first arcuate portion and a second arcuate portion that extend from opposing ends of the central portion, each of the first arcuate portion and the second arcuate portion extending from a respective end of the central portion to a respective one of the first end portion and the second end portion along a respective arcuate path which is sized to wrap around an eye socket of the subject when the face of the subject is engaged with the patient interface. The first end portion of the contact surface may be provided at an end of the first arcuate portion which is distal from the central portion, and the second end portion of the contact surface may be provided at an end of the second arcuate portion which is distal from the central portion. Additionally or alternatively, the first arcuate portion and the second arcuate portion may be arranged such that a gap is provided between the first end portion and the second end portion, wherein, when the face of the subject engages the patient interface, the nose of the subject can be accommodated within the gap without being contacted by the patient interface.

In the ophthalmic imaging instrument of the first example aspect or any of the above example embodiments, the patient interface may be orientated such that, when the subject leans in from an upright seated position to engage with the patient interface, the head of the subject has a downward inclination which is greater (e.g. by 10 to 20 degrees, and preferably by 12 to 17 degrees) than in the upright seated position.

Additionally or alternatively, the image acquisition apparatus may, in accordance with a further example embodiment, comprise an objective which is movable along a movement axis towards and away from the eye while the face of the subject is engaged with the patient interface, and the patient interface may be arranged to fix the orientation of the head relative to the movement axis such that the objective can be moved along the movement axis to a position for acquiring the image of the eye. The ophthalmic imaging instrument of the further example embodiment may be one of a widefield ophthalmic imaging instrument and an ultra-widefield ophthalmic imaging instrument. In a variant of the ophthalmic imaging instrument of the further example embodiment, the ophthalmic imaging instrument may further comprise: a base, on which the image acquisition apparatus is slidably mounted; and a gantry which extends from the base and supports the patient interface, wherein the image acquisition apparatus is slidable relative to the base to move the objective along the movement axis towards and away from the eye when the face of the subject is engaged with the patient interface. In a variant of the ophthalmic imaging instrument of the further example embodiment the objective may be moveable in a lateral direction such that the objective can be moved between left and right eye imaging positions and/or aligned laterally for imaging the subject's eye.

Additionally or alternatively, the ophthalmic imaging instrument of the further example embodiment or the variant thereof may further comprise a stereo camera ranging system for measuring a distance between the objective and the eye, the stereo camera ranging system comprising a first camera and a second camera arranged to image the eye from different locations relative to the objective, and the patient interface may be arranged to fix the orientation of the head relative to the ophthalmic imaging instrument such that each of the first camera and the second camera has a respective view of the eye which is unobstructed by a facial feature of the subject when the face of the subject is engaged with the patient interface.

In the ophthalmic imaging instrument of the first example aspect or any of the above example embodiments and variants, the patient interface may be operable to fix the orientation of the head relative to the ophthalmic imaging instrument in a first relative orientation for imaging a right eye of the subject by the ophthalmic imaging instrument, and a second relative orientation for imaging a left eye of the subject by the ophthalmic imaging instrument, the second relative orientation being different to the first relative orientation. In this case, and where the ophthalmic imaging instrument has the features of the variant of the further example embodiment set out above, the gantry may comprise a curved guide rail, and the patient interface may be slidably mounted on the curved guide rail so as to be moveable between: a first position on the guide rail, at which the patient interface is arranged to fix the orientation of the head relative to the ophthalmic imaging instrument in the first relative orientation for imaging the right eye of the subject; and a second position on the guide rail (which is different from the first position), at which second position the patient interface is arranged to fix the orientation of the head relative to the ophthalmic imaging instrument in the second relative orientation for imaging the left eye of the subject. The ophthalmic imaging instrument may further comprise a drive mechanism having a Bowden cable which is arranged to move the patient interface between the first position on the curved guide rail and the second position on the curved guide rail.

In the ophthalmic imaging instrument of the first example aspect or any of the above example embodiments and variants, the patient interface may comprise at least one compressible contact member which is arranged to contact the first portion of the face and each second portion of the face when the face of the subject engages with the patient interface. The image acquisition apparatus may be an optical coherence tomography (OCT) image acquisition apparatus arranged to acquire at least one of an OCT image of a retina of the eye, an OCT angiography image of the retina, or an OCT image of an anterior segment of the eye, for example.

There is provided, in accordance with a second example aspect herein, a patient interface attachable to an ophthalmic imaging instrument, wherein the patient interface, when attached to the ophthalmic imaging instrument and engaged with a face of a subject, is arranged to fix a position and an orientation of a head of the subject relative to the ophthalmic imaging instrument by contacting a first portion of the face overlying a frontal bone of the head and a respective left and right portions of the face overlying each zygomatic bone of the head, such that the ophthalmic imaging instrument can acquire an image of the eye.

In some example embodiments, the patient interface may be arranged to avoid contacting a nose of the subject and a third portion of the face overlying a maxilla of the head when the face of the subject is engaged with the patient interface. The patient interface may have an elongate (and preferably continuous) contact surface comprising a central portion arranged to contact the first portion of the face when the face of the subject is engaged with the patient interface, the contact surface extending to (and preferably no lower than) each of a first end portion of the contact surface and a second end portion of the contact surface, the first end portion and the second end portion being arranged to respectively contact the second portion of the face when the face of the subject is engaged with the patient interface. The elongate and preferably continuous contact surface may be a surface of a single moulding or extrudate, for example. Furthermore, the end portions of the contact surface may be immoveable (or fixed) with respect to the central portion of the contact surface, the patient interface being rigid. The patient interface may alternatively be configured to distort, for example flex and/or plastically indent, when engaged by the face of the subject, with the end portions of the contact surface being moved with respect to the central portion of the contact surface by the subject's face; in this case, the shape of the contact surface may be changeable only by pressure applied to parts of the contact surface.

The elongate contact surface further may comprise a first arcuate portion and a second arcuate portion that extend from opposing ends of the central portion, each of the first arcuate portion and the second arcuate portion extending from a respective end of the central portion to a respective one of the first end portion and the second end portion along a respective arcuate path which is dimensioned to wrap around an eye socket of the subject when the face of the subject is engaged with the patient interface. The first end portion of the contact surface may be provided at an end of the first arcuate portion which is distal from the central portion, and the second end portion of the contact surface may be provided at an end of the second arcuate portion which is distal from the central portion. Additionally or alternatively, the first arcuate portion and the second arcuate portion may be arranged such that a gap is provided between the first end portion and the second end portion, wherein, when the face of the subject engages the patient interface, the nose of the subject can be accommodated within the gap without being contacted by the patient interface.

There is provided, in accordance with a third example aspect herein, an ophthalmic imaging instrument for imaging an eye of a subject, the ophthalmic imaging instrument having attached thereto the patient interface of the second example aspect or any of its example embodiments set out above, the ophthalmic imaging instrument comprising an image acquisition apparatus arranged to acquire an image of the eye while the face of the subject is engaged with the patient interface.

In the ophthalmic imaging instrument of the third example aspect, the patient interface may be orientated such that, when the subject leans in from an upright seated position to engage with the patient interface, the head of the subject has a downward inclination which is greater (e.g. by 10 to 20 degrees, and preferably by 12 to 17 degrees) than in the upright seated position.

Additionally or alternatively, in the ophthalmic imaging instrument of the third example aspect, the image acquisition apparatus may, in accordance with an example embodiment, comprise an objective which is movable along a movement axis towards and away from the eye while the face of the subject is engaged with the patient interface, and the patient interface may be arranged to fix the orientation of the head relative to the movement axis such that the objective can be moved along the movement axis to a position for acquiring the image of the eye. The ophthalmic imaging instrument may be one of a widefield ophthalmic imaging instrument and an ultra-widefield ophthalmic imaging instrument. The ophthalmic imaging instrument may further comprise: a base, on which the image acquisition apparatus is slidably mounted; and a gantry which extends from the base and supports the patient interface, wherein the image acquisition apparatus is slidable relative to the base to move the objective along the movement axis towards and away from the eye when the face of the subject is engaged with the patient interface. In a variant of the ophthalmic imaging instrument of the further example embodiment the objective may be moveable in a lateral direction such that the objective can be moved between left and right eye imaging positions and/or aligned laterally for imaging the subject's eye.

Additionally or alternatively, the ophthalmic imaging instrument of the third example aspect may further comprise a stereo camera ranging system for measuring a distance between the objective and the eye, the stereo camera ranging system comprising a first camera and a second camera arranged to image the eye from different locations relative to the objective, and the patient interface may be arranged to fix the orientation of the head relative to the ophthalmic imaging instrument such that each of the first camera and the second camera has a respective view of the eye which is unobstructed by a facial feature of the subject when the face of the subject is engaged with the patient interface.

In the ophthalmic imaging instrument of the third example aspect or any of its above example embodiments, the patient interface may be operable to fix the orientation of the head relative to the ophthalmic imaging instrument in a first relative orientation for imaging a right eye of the subject by the ophthalmic imaging instrument, and a second relative orientation for imaging a left eye of the subject by the ophthalmic imaging instrument, the second relative orientation being different to the first relative orientation. In this case, and where the ophthalmic imaging instrument comprises a base, on which the image acquisition apparatus is slidably mounted; and a gantry which extends from the base and supports the patient interface, wherein the image acquisition apparatus is slidable relative to the base to move the objective along the movement axis towards and away from the eye when the face of the subject is engaged with the patient interface, the gantry may comprise a curved guide rail, and the patient interface may be slidably mounted on the curved guide rail so as to be moveable between: a first position on the guide rail, at which the patient interface is arranged to fix the orientation of the head relative to the ophthalmic imaging instrument in the first relative orientation for imaging the right eye of the subject; and a second position on the guide rail (which is different from the first position), at which second position the patient interface is arranged to fix the orientation of the head relative to the ophthalmic imaging instrument in the second relative orientation for imaging the left eye of the subject. The ophthalmic imaging instrument may further comprise a drive mechanism having a Bowden cable which is arranged to move the patient interface between the first position on the curved guide rail and the second position on the curved guide rail.

In the ophthalmic imaging instrument of the third example aspect or any of its above example embodiments, the patient interface may comprise at least one compressible contact member which is arranged to contact the first portion of the face and each second portion of the face when the face of the subject engages with the patient interface. The image acquisition apparatus may be an OCT image acquisition apparatus arranged to acquire at least one of an OCT image of a retina of the eye, an OCT angiography image of the retina, or an OCT image of an anterior segment of the eye, for example.

There is provided, in accordance with a fourth example aspect herein, a method of imaging an eye of a subject with an ophthalmic imaging instrument, wherein the ophthalmic imaging instrument comprises a patient interface with which a face of a head of the subject is to engage during the imaging of the eye of the subject, the method comprising: engaging the face of the head of the subject with the patient interface; fixing the subject's head in an orientation relative to the ophthalmic imaging instrument by contacting an upper portion of the face overlying a frontal bone of the head and respective left and right portions of the face overlying each zygomatic bone of the head when the face of the subject is engaged with the patient interface; and acquiring an image of the eye while the face of the head of the subject is engaged with the patient interface.

In some example embodiments the ophthalmic imaging instrument may comprise an objective which is moveable along a movement axis towards and away from the eye while the face of the subject is engaged with the patient interface, wherein fixing the subject's head may comprise fixing the orientation of the head relative to the movement axis and moving the objective along the movement axis to an imaging position for acquiring the image of the eye.

Additionally or alternatively, the method may further comprise imaging both eyes of the subject, wherein: fixing the patient's head in the orientation relative to the ophthalmic imaging instrument comprises: fixing the head in a first relative orientation for imaging a first eye of the subject by the ophthalmic imaging instrument; and fixing the head in a second relative orientation for imaging a second eye of the subject by the ophthalmic imaging instrument.

In some example embodiments fixing the head in the second relative orientation may comprise moving the patient interface from a first position to a second position. Moving the patient interface from the first position to the second position may comprise sliding the patient interface along an arcuate path such that when the patient interface is in the first position the patient interface is orientated at a first angle relative to the movement axis of the objective and when the patient interface is in the second position the patient interface is orientated at a second angle relative to the movement axis of the objective wherein the first angle is different to the second angle.

In some example embodiments, the method may further comprise moving the objective from a retracted position to the imaging position when the subject's head is fixed in the orientation relative to the ophthalmic imaging instrument. Moving the objective from the retracted position to the imaging position may comprise acquiring images of an anterior region of the eye, determining a distance between the anterior region of the eye and the ophthalmic imaging instrument and/or objective based on the acquired images and moving the objective to the imaging position based on the determined distance. The anterior region of the eye may be, for example, the subject's pupil. The method may comprise using a camera to acquire images of the anterior region of the eye and monitoring the acquired images for obstruction of a view of the anterior region of the eye by an anatomical feature of the face. The method may further comprise changing orientation of the patient interface if there is an obstruction in the images acquired by the camera.

In some example embodiments, the method may comprise monitoring a separation between the objective and the face of the subject whilst moving the objective from the retracted position to the imaging position and stopping moving the objective from the retracted position to the imaging position if a collision is predicted and/or alerting a user of the ophthalmic imaging instrument that a collision has bene predicted.

In some example embodiments, engaging the face of the head of the subject with the patient interface may comprise angling the head of the subject downwardly before engaging the patient interface such that the head of the subject has a downward inclination which is greater (e.g. by 10 to 20 degrees, and preferably by 12 to 17 degrees) than in the upright seated position.

### [Brief Description of the Drawings]

Example embodiments will now be explained in detail, by way of non-limiting example only, with reference to the accompanying figures described below. Like reference numerals appearing in different ones of the figures can denote identical or functionally similar elements, unless indicated otherwise.
Figure 1 is a side-view of an ophthalmic imaging instrument according to an example embodiment herein.
Figure 2 is an end view of the ophthalmic imaging instrument of the example embodiment herein, viewed from the end at which the subject is disposed during imaging.
Figure 3 is a top view of the gantry and patient interface of the example embodiment.
Figure 4 is an end view of the ophthalmic imaging instrument of the example embodiment herein, viewed from the end at which the subject is disposed during imaging, with the patient interface located in a first position.
Figure 5 is an end view of the ophthalmic imaging instrument of the example embodiment herein, viewed from the end at which the subject is disposed during imaging, with the patient interface located in a second position.
Figure 6 is a perspective view of the gantry and patient interface of the ophthalmic imaging instrument of the example embodiment.
Figure 7 is another perspective view of the gantry and patient interface of the ophthalmic imaging instrument of the example embodiment, showing a cross-section of the gantry and the patient interface.
Figure 8 is an end view of the patient interface of the example embodiment, viewed from the end at which the subject is disposed during imaging.
Figure 9 is a side view of the patient interface of the example embodiment.
Figure 10 is a top view of the patient interface of the example embodiment.
Figure 11 is a flow chart outlining a method of imaging an eye of a subject with an ophthalmic imaging instrument.
Figure 12 is a flow chart outlining a method of imaging both eyes of a subject with an ophthalmic imaging instrument.

### [Detailed Description of Example Embodiments]

Conventional patient interfaces typically include a forehead rest and a chin rest to help steady the subject's head during imaging, whilst allowing the head to rotate about these pivot points, and the subject is normally requested to keep as still as possible to minimise such rotation. However, this is often not sufficient, and patients' involuntary movements that often occur during imaging lead to lower quality images than might otherwise be achieved. In view of these shortcomings of conventional patient interfaces, the inventors have devised a patient interface which is arranged to suppress the rotational degree of freedom in the head movement noted above, and thus fix the orientation of the subject's head relative to the ophthalmic imaging instrument. The patient interface of example embodiments described herein does this by contacting not only a first portion of the face overlying the frontal bone of the head (thus contacting the subject's forehead, for example) but also a respective second portion of the face overlying each zygomatic bone (i.e. cheek bone) of the head when the face of the subject engages with the patient interface. The patient interface can thus securely contact the subject's head at three different contact areas, as the soft tissue overlaying the frontal bone and the cheek bones is relatively thin. Furthermore, these contact areas are spaced widely apart and, as such, provide superior stability of the head during the imaging of the eye. These example embodiments will now be described in more detail with reference to the accompanying drawings.

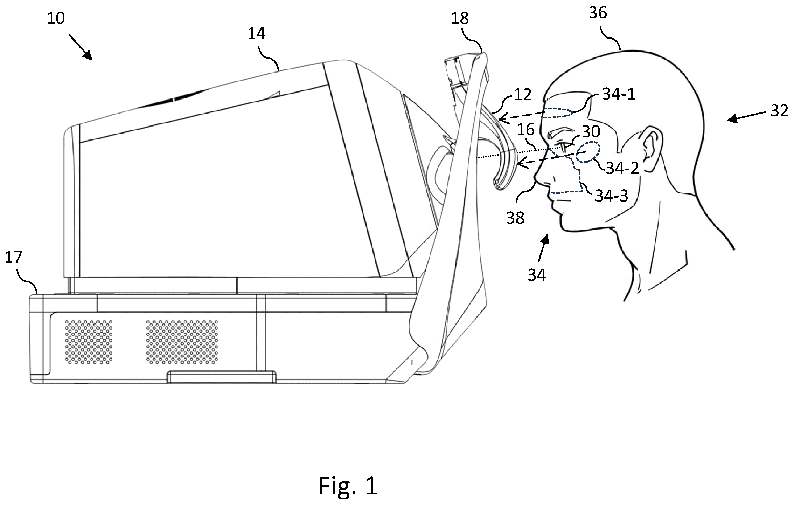

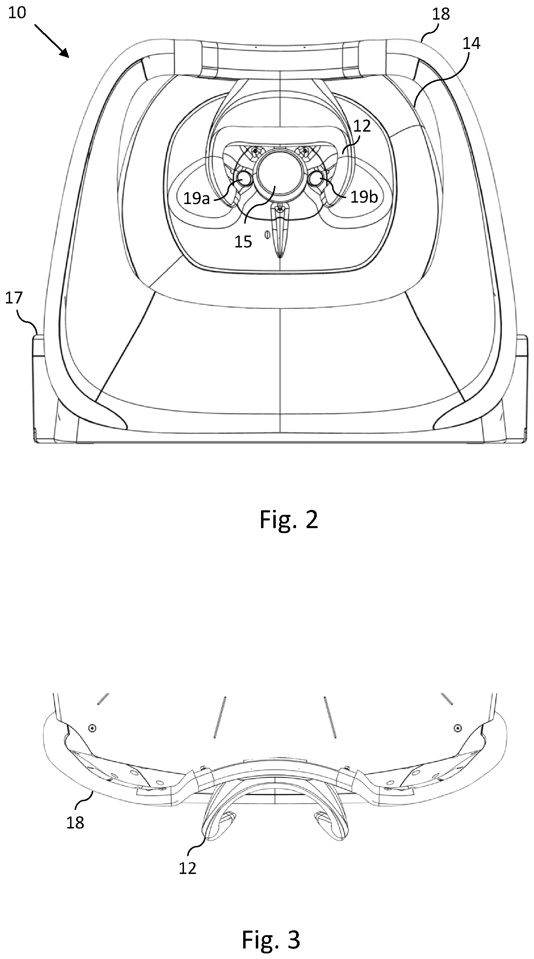

Figure 1 is a schematic side view of an ophthalmic imaging instrument 10 according to an example embodiment, which is configured to image an eye 30 of a (human, typically adult) subject 32. Figure 2 is an end view of the ophthalmic imaging instrument 10, in this case taken from the perspective of the subject 32. Figure 3 is a top view of the ophthalmic imaging instrument 10. The ophthalmic imaging instrument 10 comprises a patient interface 12 (which may also be referred to as a head support or head stabilizer), with which the face 34 of the subject 32 is to engage during the imaging of the eye 30, such that the patient interface 12 helps to keep the head 36 steady during the imaging. The ophthalmic imaging instrument 10 further comprises an image acquisition apparatus 14 arranged to acquire one or more images of the eye 30 while the face 34 of the subject 32 is engaged with the patient interface 12.

The image acquisition apparatus 14 can take various forms. For example, the image acquisition apparatus 14 may, as in the present example embodiment, be an optical coherence tomography (OCT) imaging apparatus in the form of a swept-source OCT (SS-OCT) imaging apparatus having well-known components, such as a light beam generator, a scanning system, an interferometer, a light detector, OCT data processing hardware, and an objective lens system (or "objective"). The scanning system may be arranged to perform a one- and/or two-dimensional point-scan of a light beam across the retina of the eye 30 and collect light which has been scattered by the retina during the point scan. The image acquisition apparatus 14 may thus acquire A-scans at respective scan locations that are distributed across a surface of the retina, by sequentially illuminating the scan locations with the light beam, one scan location at a time, and collecting at least some of the light scattered by the retina at each scan location. The image acquisition apparatus 14 may be arranged to acquire OCT images in the form of B-scans by performing the point-scan to acquire successive A-scans along, for example, a straight line. However, the image acquisition apparatus 14 may alternatively be arranged to acquire the B-scans by the scanning system performing line-scans, using hardware well-known to those versed in the art. More generally, the image acquisition apparatus 14 may be arranged to acquire OCT images in the form of B-scans or C-scans by performing point-scans or a line-scans using predetermined scanning patterns well-known to those versed in the art (e.g. a spiral scan), or by employing a full-field set-up.

The image acquisition apparatus 14 may, however, be another kind of Fourier-domain OCT (FD-OCT) imaging apparatus, such as a spectral-domain OCT (SD-OCT) imaging apparatus, or may alternatively be a time-domain OCT (TD-OCT) imaging apparatus. However, the image acquisition apparatus 14 is not so limited and may be any other type of ophthalmic image acquisition apparatus for imaging the posterior segment of the eye 30, such as a scanning laser ophthalmoscope (SLO) or a fundus camera, for example. Furthermore, the image acquisition apparatus 14 need not be limited to imaging the posterior of the eye 30 and may alternatively or additionally be arranged to acquire an OCT image or other type of image (e.g. reflectance) of the anterior segment of the eye 30.

The image acquisition apparatus 14 may, as in the present example embodiment, be a widefield (WF) image acquisition apparatus, which is operable to acquire a WF image of the fundus of the eye 30. A WF image of the fundus is defined as a single-capture image, centred on the fovea of the eye 30, which captures retinal anatomic features in the mid-periphery that are posterior to the vortex vein ampulla, in all four quadrants (i.e. superior, inferior, nasal and temporal) of the eye 30. A WF image acquisition apparatus can thus acquire a single-capture image which covers a region of the retina extending from the fovea up to the posterior edge of vortex vein ampulla, and is defined to have a field of view (FoV) between 60 and 100 degrees. Here, the FoV is expressed in terms of the eye-angle, which is the angle subtended by the imaged retinal region at the spherical centre of the eye 30, i.e. the point of intersection between the vertical diameter of the eyeball and the visual axis.

The image acquisition apparatus 14 may alternatively be provided in the form of an ultra-widefield (UWF) image acquisition apparatus, which has a larger FoV than a WF apparatus and is operable to acquire an UWF image of the ocular fundus covering a larger proportion of the ocular fundus. An UWF image of the ocular fundus is defined as a single-capture image, centred on the fovea of the eye 30, which captures retinal anatomic features in the far periphery that are anterior to the vortex vein ampulla in all four quadrants. An UWF image acquisition apparatus can thus acquire an UWF image being a single-capture image which covers a region of the retina extending from the fovea to the anterior edge of vortex vein ampulla and beyond to pars plana, and is defined to have a FoV (expressed in terms of the eye-angle) between 110 degrees and 220 degrees. By way of an example, the Optos Daytona^{™} is capable of UWF images (so -called Optomap^{™} images) covering up to 200 degrees of the fundus (or approximately 82% of the retina) in a single capture.

It should be noted that the techniques described herein are not limited in their applicability to WF and UWF image acquisition apparatuses but are also applicable to ophthalmic image acquisition apparatuses having a smaller FoV.

The image acquisition apparatus 14 may, as in the present example embodiment, comprise an objective 15, which is movable along a movement axis 16 towards and away from the eye 30 while the face 34 of the subject 32 is engaged with the patient interface 12. In this case, the patient interface 12 may be arranged to fix the orientation of the head 36 relative to the movement axis 16 such that the objective 15 can be moved along the movement axis 16 from a retracted position to an imaging position which is suitable for acquiring the image of the eye 30, whilst remaining more than a predetermined distance away from the nose 38 and the brow of the subject 32, to provide sufficient clearance between the objective 15 and the subject's face 34. The retracted position may be a position in which the objective 15 is retracted along the movement axis 16, away from the patient interface 12 such that when the subject 32 is engaged with the patient interface 12 the objective 15 is located further from the subject 32 in the retracted position than in the imaging position. Configuring the patient interface 12 to orientate the head 36 in this way may be particularly beneficial for WF and UWF implementations of the image acquisition apparatus 14, whose objective 15 is typically required to move into close proximity of the eye 30 in order to enable WF/UWF imaging, and is therefore susceptible to coming into contact with facial features (such as the nose 38 or the brow of the subject 32) unless the head 36 is appropriately orientated relative to the movement axis 16. Contact between the objective 15 and the subject 32 may cause the subject 32 to move and possibly disengage from the patient interface 12, thereby requiring the patient alignment procedure to be repeated.

The ophthalmic imaging instrument 10 may, as in the present example embodiment, further comprise a stereo camera ranging system for measuring a distance between the objective 15 and the eye 30, the stereo camera ranging system comprises a first camera 19a and a second camera 19b arranged to image the eye 30 from different locations relative to the objective 15 (e.g. on opposite sides of the objective 15). For example, the first and second cameras 19a, 19b may image an anterior region of the eye 30 such as the pupil of the eye 30. In this case, the patient interface 12 is preferably arranged to fix the orientation of the head 36 relative to the ophthalmic imaging instrument 10 to fulfil the further requirement that each of the first camera 19a and the second camera 19b has a respective view of the eye 30 that is unobstructed by an obstruction such as a facial feature (e.g. the nose or the brow) of the subject 32 or by the patient interface 12 itself, particularly when the objective 15 is at or approaching the position at which the image acquisition apparatus 14 can acquire the WF image of the subject's retina. Taking this additional constraint into account when setting the orientation of the patient interface 12 relative to the remainder of the ophthalmic imaging instrument 10 may help reduce the average duration of imaging sessions and thus increase patient throughput, by reducing the incidence of failures of the stereo camera ranging system that are caused by obstruction of the eye 30 by the nose or brow of the subject 32 in one or both of the stereo images acquired by the stereo camera ranging system.

The stereo ranging system may be arranged to monitor images captured by the first camera 19a and second camera 19b for obstructions that obscure the view of the eye 30, such as the view of the pupil of the eye 30. For example, as the objective 15 is actuated from the retracted position to the imaging position one or more anatomical features of the subject's face 34 such as the nose or brow may at least partially obscure the views of the first camera 19a and/or the second camera 19b. The stereo ranging system may be arranged to monitor the images captured by the stereo ranging system for obstructions and if an obstruction is detected in the images the stereo ranging system may be arranged to perform one or more of: stop movement of the objective 15, generate an alert to a clinician that the cameras 19a, 19b have been obstructed or retract the objective 15 until the obstruction has been removed.

The ophthalmic imaging instrument 10 may, as in the present example embodiment, further comprise a base 17, on which the image acquisition apparatus 14 is slidably mounted so as to move along the movement axis 16, as well as a gantry 18 which extends from the base 17 and supports the patient interface 12. The image acquisition apparatus 14 is moveable relative to the base 17 such that that the objective 15 can move along the movement axis 16 towards and away from the eye 30 when the face 34 of the subject 32 is engaged with the patient interface 12. The image acquisition apparatus 14 may also be moveable relative to the base 17 in a lateral direction relative to the movement axis 16. This configuration advantageously allows any moving components of the image acquisition apparatus 14, such as a cooling fan which may be provided to cool the electrical components of the image acquisition apparatus 14, to be encased within an enclosure that is set apart from the gantry 18 and the patient interface 12 supported thereby, making it easier to shield the eye 30 (e.g. using the enclosure and a seal between any parts of the enclosure that meet in the vicinity of the eye 30) from air currents generated within the image acquisition apparatus 14 and thus prevent these air currents from reaching and consequently irritating or drying the eye 30 during imaging. Supporting the patient interface 12 from the gantry 18, such that the subject's face 34 is set apart from the image acquisition apparatus 14 when the face 34 is engaged with the patient interface 12, may provide the further advantage of allowing the clinician to access the face 34 more easily to perform lid-lifting, presentation of a hand-held fixation target and the like during imaging.

The patient interface 12 may, as in the present example embodiment, be switchable between a first configuration for fixing the orientation of the head 36 relative to the ophthalmic imaging instrument 10 in a first relative orientation which is suitable for the imaging of the right eye of the subject 32 by the ophthalmic imaging instrument 10, and a second configuration for fixing the orientation of the head 36 relative to the ophthalmic imaging instrument 10 in a second relative orientation which is suitable for the imaging of the left eye of the subject 32 by the ophthalmic imaging instrument 10, where the second relative orientation is different to the first relative orientation. The ability to switch the configuration of the patient interface 12 in this way, for example by moving the patient interface 12 between a first rest position and a second rest position, may allow the orientation of the head 36 to be set optimally for the imaging of each eye of the subject 32 by the ophthalmic imaging instrument 10.

The first relative orientation of the head 36 that is suitable for imaging the right eye of the subject 32 may be an orientation in which the subject's head 36 is rotated counterclockwise from a forward-facing orientation such that the head 36 is facing to the left of the movement axis 16 of the objective 15 (from the perspective of the subject 32). Rotating the subject's head 36 in a counterclockwise direction has the effect of moving the subject's nose and brow away from the movement axis 16 of the objective 15 thereby enabling the objective 15 to be positioned at the imaging position close to the subject's eye 30 without being obstructed by the nose and/or brow of the head 36. The head 36 may be orientated by between approximately 10 degrees and 40 degrees relative to the movement axis 16 of the objective 15 when the head 36 is fixed in the first orientation by the patient interface 12.

The second relative orientation of the head 36 that is suitable for imaging the left eye of the subject 32 may be an orientation in which the subject's head 36 is rotated clockwise from a forward-facing orientation such that the head 36 is facing to the right of the movement axis 16 of the objective 15 (from the perspective of the subject 32). Rotating the subject's head in a clockwise direction has the effect of moving the subject's nose and brow away from the optical axis of the objective thereby enabling the objective 15 to be positioned at the working distance from the subject's eye 30 without being obstructed by the nose and/or brow of the head 36. The head 36 may be orientated by between approximately 10 degrees and 40 degrees relative to the movement axis 16 of the objective 15 when the head 36 is fixed in the second orientation by the patient interface 12.

Furthermore, rotating the subject's nose and brow away from the objective 15 allows the subject's head 36 to be orientated relative to the ophthalmic imaging instrument 10 such that each of the first camera 19a and the second camera 19b has a respective view of the eye 30 that is unobstructed by a facial feature (e.g. the nose or the brow) of the subject 32.

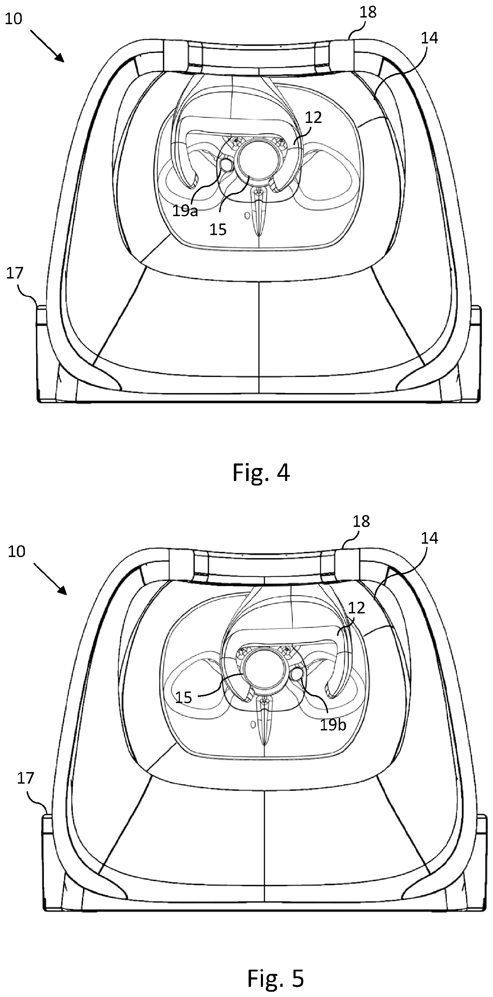

Where the ophthalmic imaging instrument 10 comprises the base 17 and the gantry 18 as described above, the gantry 18 may, as illustrated in Figure 4, comprise a curved guide rail 18a, and the patient interface 12 may be slidably mounted on the curved guide rail 18a, via a part 12a thereof that is arranged to engage and slide along the curved guide rail 18a (as shown in Figure 5), so as to be moveable between (i) a first position on the guide rail 18a, at which the patient interface 12 is arranged to fix the orientation of the head 36 relative to the ophthalmic imaging instrument 10 in the first relative orientation for imaging the subject's right eye, and (ii) a second position on the guide rail 18a which is different from the first position, at which second position the patient interface 12 is arranged to fix the orientation of the head 36 relative to the ophthalmic imaging instrument 10 in the second relative orientation for imaging the subject's left eye. In this case, the ophthalmic imaging instrument 10 may further comprise a drive mechanism arranged to move the patient interface 12 between the first position on the curved guide rail 18a and the second position on the curved guide rail 18a. For example, the drive mechanism may have a Bowden cable 18b, and the Bowden cable 18b may be arranged to push or pull the patient interface 12 between the first position on the curved guide rail 18a and the second position on the curved guide rail 18a. Alternatively, the patient interface 12 may be manually moved between the first position and the second position.

Figure 4 is an end view of the ophthalmic imaging instrument 10 taken from the perspective of the subject 32, with the patient interface 12 in the first configuration or first position. As shown in Figure 4, when the patient interface 12 is in the first position the patient interface 12 is moved along the gantry 18 to the first position. In the example shown, the first position is a position in which the patient interface 12 is moved along the gantry 18 from a straight-on position (as shown in Figure 2) to a left position. When the patient interface 12 is moved to the left position it is positioned such that when the subject 32 engages the patient interface 12 the subject's head 36 is in the first relative orientation that is suitable for imaging the subject's right eye. The orientation of the patient interface 12 in the first position relative to the ophthalmic imaging device 10 may be the same orientation as the subject's head 36 when the head 36 is in the first relative orientation.

Figure 5 is an end view of the ophthalmic imaging instrument 10 taken from the perspective of the subject 32 with the patient interface 12 in the second configuration or second position. As shown in Figure 5, when the patient interface 12 is in the second position the patient interface 12 is moved along the gantry 18 from the straight-on position (as shown in Figure 2) to the second position. In the example shown, the second position is a position in which the patient interface 12 is moved along the gantry 18 to a right position. When the patient interface 12 is moved to the right position it is position such that when the subject 32 engages the patient interface 12 the subject's head 36 is in the second relative orientation that is suitable for imaging the subject's left eye. The orientation of the patient interface 12 in the second position relative to the ophthalmic imaging device 10 may be the same orientation as the subject's head 36 when the head 36 is in the second relative orientation.

The use of a curved guide rail 18a in this way may provide a simple and reliable mechanism, with a relatively low component count, for switching the configuration of the patient interface 12 between the first configuration and the second configuration. Furthermore, by appropriately setting the curvature of the guide rail 18a, such that it presents a concave section from the perspective of the subject 32, the right eye and the left eye of the subject 32 may be presented to the image acquisition apparatus 14 at a similar position relative to the image acquisition apparatus 14 when the patient interface 12 is at the first position on the guide rail 18a and the second position on the guide rail 18a, respectively. For example, the curvature of the guide rail 18a may follow an arcuate path that is substantially centred at a point corresponding to the axis of rotation of the subject's head 36. In other words, the arcuate path that the patient interface 12 follows as it moves between the first and second position is substantially the same path that the subject's face 34 would follow if the subject 32 were to remain engaged with the patient interface 12 as it moved between the first and second positions. Accordingly, relatively little horizontal movement of the objective 15 may be required to bring the objective 15 into a lateral alignment with each eye, thus allowing both the subject's eyes to be examined in relatively quick succession. A further benefit may be that the subject 32 may simply rotate their head 36 in a natural way after having had one of his/her eyes imaged by the ophthalmic imaging instrument 10, in order to change the orientation of the head 36 from the first orientation to the second orientation for imaging the remaining eye. Relatively little adjustment of the subject's body may therefore be required to image both eyes, which may reduce the duration of the imaging session.

It should be noted, however, that the patient interface 12 may be switchable between the first configuration and the second configuration other than by using a curved guide rail 18a, for example by employing a relatively complex arrangement of linkages which substantially reproduces the movement of the patient interface 12 as it slides between the first position and second position as described above. As a further alternative, the patient interface 12 may be switchable between the first configuration and the second configuration by simply pivoting about a point proximate to the subject's forehead when the subject's face 34 is engaged with the patient interface 12. However, in this case, the subject 32 would be required to move their head 36 laterally (as well as rotating it), with a corresponding lateral movement of the objective 15 being required, when the patient interface 12 switches between the first configuration and the second configuration.

The patient interface 12 may, as in the present example embodiment, be operable in a third configuration for fixing the orientation of the head 36 relative to the ophthalmic imaging instrument 10 in a third relative orientation which is suitable for the imaging of both the right eye and left eye of the subject 32 by the ophthalmic imaging instrument 10. The third configuration may be the straight-on position as shown in Figure 2. In the third configuration the patient interface may fix the orientation of the head 36 relative to the ophthalmic imaging instrument 10 so that the head 36 is orientated in a manner such that it is aligned with the movement axis 16 of the objective 15 as shown in Figure 2. When the patient interface 12 fixes the orientation of the head 36 in the third position, the user is looking directly at the objective 15. The objective 15 may be actuated laterally such that the objective 15 can be moved between a left eye and right eye imaging position without changing the orientation of the head 36.

In some example embodiments, the ophthalmic imaging instrument 10 may comprise a patient interface 12 that is fixed in the third configuration or straight-on position such that the orientation of the user's head 36 is fixed in the third relative orientation as described above. Furthermore, in some example embodiments, the ophthalmic imaging instrument 10 may comprise a patient interface 12 that is moveable between either: the first and second configurations or the first, second and third configurations.

Regarding the form of the patient interface 12, this differs from conventional patient interfaces in some important respects. Conventional patient interfaces typically include a forehead rest and a chin rest to help steady the subject's head 36 during imaging, whilst allowing the head 36 to rotate about these pivot points, and it is left to the subject 32 to minimise such rotation. However, the patient interface 12 of the present example embodiment is arranged to suppress this degree of freedom in the head movement and thus fix the orientation of the subject's head 36 relative to the ophthalmic imaging instrument 10. The patient interface 12 does this by contacting not only a first portion 34-1 (also referred to as an upper portion) of the face 34 overlying the frontal bone of the head 36 (thus contacting the subject's forehead, for example) but also a respective second portion 34-2 (also referred to as left and right portions) of the face 34 overlying each zygomatic bone (i.e. cheek bone) of the head 36 when the face 34 of the subject 32 engages with the patient interface 12. The patient interface 12 can thus securely contact the subject's head 36 at three different contact areas (or anchor points), as the soft tissue overlaying the frontal bone and the cheek bones is relatively thin. Furthermore, these contact areas are spaced widely apart and, as such, provide superior stability of the head 36 during the imaging of the eye 30. The patient interface 12 may also provide support to the subject's face 34 such that the subject 32 may comfortably maintain engagement with the patient interface 12 without the requirement of an additional chin rest to provide support to the subject's head 36.

The patient interface 12 is shaped to conform with the face 34, having a generally concave form to accommodate the face 34. The patient interface 12 may, as in the present example embodiment, be shaped to avoid contacting the nose 38 of the subject 32, as well as a third portion 34-3 (also referred to as a lower portion) of the face 34 overlying a maxilla of the head 36 when the face 34 of the subject 32 is engaged with the patient interface 12. The patient interface 12 may thus be arranged to avoid contacting the nose 38 of the subject 32 and the surrounding parts of the face 34 that are disposed between the opposing second portions 34-2 and below the subject's eyes.

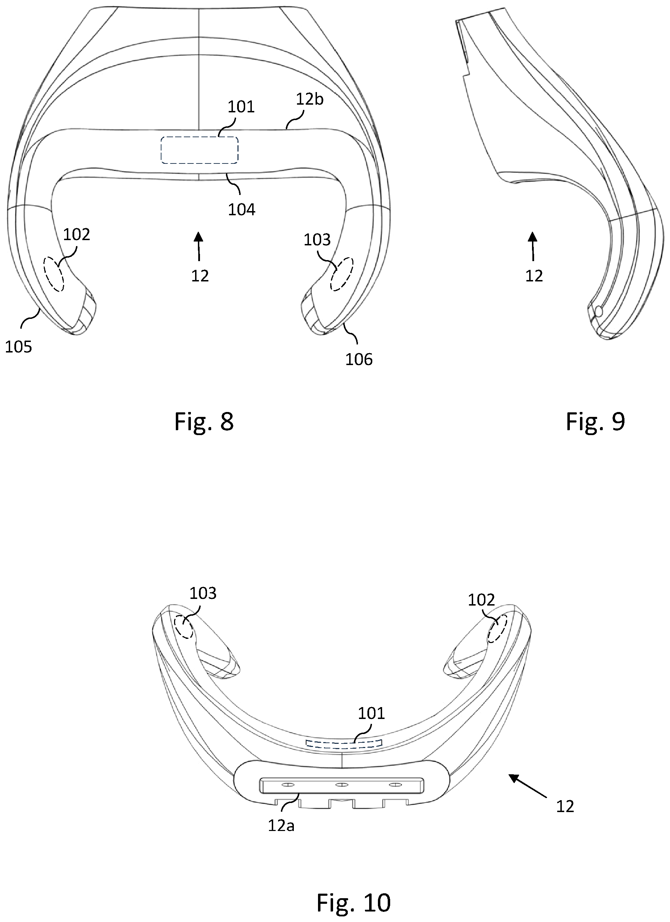

As shown in Figure 8, the patient interface 12 is generally C-shaped when viewed from the end at which the subject 32 is disposed during imaging, and has, in this view, a central (optionally straight) elongate portion 104. The central elongate portion 104 comprises a central contact portion 101 which is to engage with the first portion 34-1 of the face 34 when a subject 32 engages the patient interface 12. Respective arcuate portions 105, 106 extend downwardly and proximally from opposing ends of the central elongate portion 104. Each arcuate portion 105, 106 extends only downwardly, and proximally from opposing ends of the central elongate portion 104, along an arcuate path that is dimensioned to wrap around the eye socket of the subject 32 when the subject 32 is engaged with the patient interface 12. A gap extends between the end of each arcuate portion 105, 106 such that when a subject 32 engages the patient interface 12 the subject's nose 38 can be accommodated within the gap without being contacted by the patient interface 12.

Each arcuate portion 105, 106 comprises a respective left and right contact portion 102, 103, each of which is configured to contact a respective one of the second portions 34-2 of the face 34. The respective left and right contact portions 102, 103 of the patient interface 12 are located on respective arcuate portions 105, 106 such that the contact portions 102, 103 contact a subject's respective left and right zygomatic bones when the subject 32 engages the patient interface 12. The left contact portion 102 may be provided at an end of arcuate portion 105 which is distal from the central portion 101, and the right contact portion 103 of the contact surface may be provided at an end of arcuate portion 106 which is distal from the central portion 101. The patient interface 12 may thus have a compact configuration for securely fixing the orientation of the subject's head 36. The left and right contact portions 102, 103 are located on the patient interface 12 at a common elevation on the respective elongate arcuate portions 105, 106 of the patient interface 12 that is beneath the elevation of the central contact portion 101. As such, the left and right contact portions 102, 103 and central portion 101 are distributed in a pyramidal formation on the patient interface 12 thereby providing three contact or anchor points for the subject's face 34 to engage the patient interface 12 that are spaced apart to securely fix the orientation of the subject's head 36 when the subject 32 engages the patient interface 12.

When the subject 32 has engaged with the patient interface 12, the arcuate portions 105, 106 of the patient interface 12, when viewed from the left- or right-hand side of the subject's head 36, curve around the eye 30 of the subject 32 in an arc (not necessarily centred on the eye, however), and this curvature can be seen in Figure 9 and Figure 10. When the subject 32 has engaged with the patient interface 12, the arcuate portions 105, 106 of the patient interface 12, when viewed from above the subject's head 36, extend proximally such that each arcuate portion 105, 106 curve around the sides of the subject's face 34, and this curvature can be seen in Figure 10.

As best viewed in Figure 10, the central elongate portion 104 comprising the central contact portion 101 of the patient interface 12 may be concave such that the central contact portion 101 conforms to the shape of the upper portion 34-1 of the subject's face 34. The arcuate portions 105, 106 extend from opposing ends of the central portion 101 in a proximal direction (when viewed from above) such that each of the left and right contact portions 105, 106 are located proximally of the central contact portion 101. The patient interface 12 may comprise a continuous surface extending between each of the left and right contact portions 102, 103 with the central contact portion 101 being disposed therebetween. Subjects with relatively small heads may only contact the patient interface 12 at the upper contact portion 101 and the respective left and right contact portions 102, 103. Subjects with larger heads will also contact the patient interface 12 at the upper contact portion 101 and the respective left and right contact portions 102, 103 but they may also contact at least some additional portions of the surface extending between the respective contact portions 101, 102, 103. Beneficially, this allows subject's having a variety of head shapes and sizes to use the patient interface 12 and have their heads comfortably fixed in an orientation suitable for imaging.

The generally concave form of the patient interface 12 having the central portion 104 and the elongate arcuate portions 105, 106 described above may allow the patient interface 12 to securely hold in place heads with a variety of facial shapes, as variations in cheek bone position on the face are quite limited. Furthermore, shaping the patient interface 12 as described above, to avoid contacting the nose 38 and the surrounding parts of the subject's face 34 that are disposed between the opposing second portions 34-2 and below the subject's eyes, may allow the clinician to gain access to the subject's eye 30 to lift a lid of the eye 30 in order to facilitate imaging or to allow the clinician to insert and move a hand-held fixation target in front of one of the subject's eyes in order to vary and fix the gaze direction of the observing eye and thus also the gaze direction of the remaining eye that is being imaged by the image acquisition apparatus 14. In addition, such shape of the patient interface 12 may improve its compatibility with a broader range of facial shapes and may, in particular, prevent the usability of patient interface 12 from being impaired by variations in the shape and/or size of the nose.

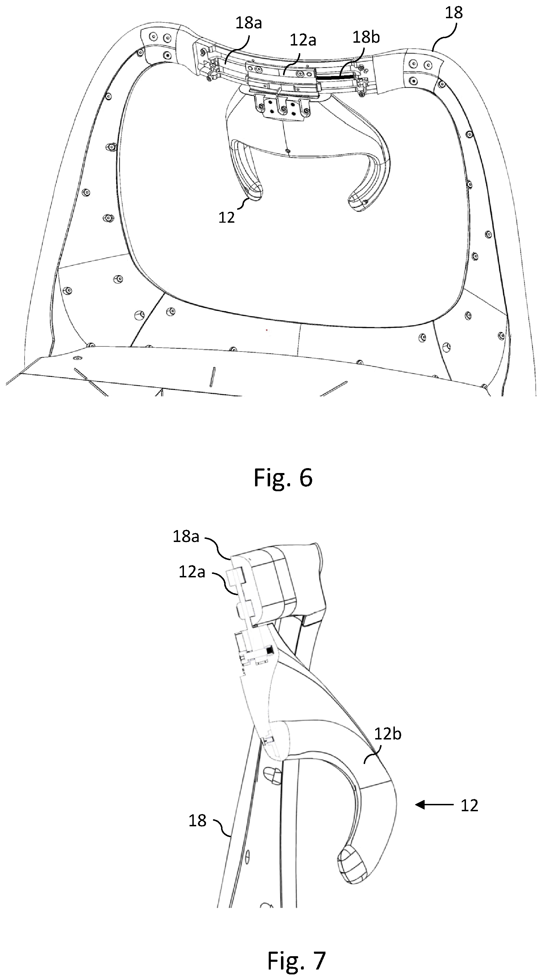

The patient interface 12 may be made and sold as a separate component, and may be attachable to an ophthalmic imaging instrument via a gantry, as described above, or in another way. The patient interface 12 of the present example embodiment is illustrated in Figures 6 to 8, and has a continuous, elongate contact surface comprising a central portion 101, which is arranged to contact the first portion 34-1 of the subject's face 34 when the face 34 is engaged with the patient interface 12. The contact surface extends continuously to each end portion 102 and 103 of the contact surface, which is arranged to contact the respective second portion 34-2 of the subject's face 34 when the face 34 is engaged with the patient interface 12. The provision of such a continuous, elongate contact surface may make it easier to wipe down the patient-facing part of the patient interface 12 between examinations of different subjects, and make the sterilization of the part more effective. The continuity of the contact surface may provide the additional benefit of increasing the contact area between the patient interface 12 and the subject's face 34, thereby helping to keep the subject's head 36 steady during imaging and making the subject 32 more comfortable by reducing the contact pressure, with the subject 32 consequently being less likely to move during imaging in order to alleviate discomfort.

The patient interface 12 may, as in the present example embodiment, comprise at least one compressible contact member 12b, with the contact surface being provided on the compressible contact member 12b so as to contact the first portion 34-1 of the face 34 and each second portion 34-2 of the face 34 when the face 34 engages with the patient interface 12. The compressible contact member 12b may be made of a medical grade silicone, for example, and may have a compressibility which is high enough to allow the patient interface 12 to accommodate different head sizes but low enough to stabilise the head 36 relative to the ophthalmic imaging instrument 10 enough to reduce or avoid motion-related image artefacts, regardless of the head size. Providing the patient interface 12 with such a compressible contact member 12b may therefore improve the versatility of the patient interface 12. However, even where the patient interface 12 is rigid and does not have a compressible contact member 12b, the patient interface 12 may be shaped to accommodate a reasonably wide range of adult head sizes. Furthermore, in some example embodiments, differently sized versions of the patient interface 14 (with or without the compressible contact member 12b) may be provided, which may be interchangeably attached to the ophthalmic imaging instrument 10 to allow it to cater for a broader range of head sizes than it might otherwise (for example, to cater for young children), providing good head stabilization although at the expense of patient throughput in some cases.

As an additional or alternative way of improving the image quality of acquired ocular images by making patients more comfortable and therefore less susceptible to moving to alleviate discomfort during imaging, the patient interface 12 may be positioned and orientated (e.g. relative to the remainder of the ophthalmic imaging instrument 10) such that, when the subject 32 leans in (i.e. forward) from an upright seated position to engage with the patient interface 12, the head 36 of the subject 32 has a downward inclination which is greater, for example by 10 to 20 degrees (preferably by 13 to 17 degrees, and more preferably by 15 degrees), than the inclination of the head 36 when the subject 32 is in the upright seated position. This may allow the subject 32 to adopt a more natural pose when engaged with the patient interface 12 than they would when engaged with the patient interface 12 not having the described position and orientation.

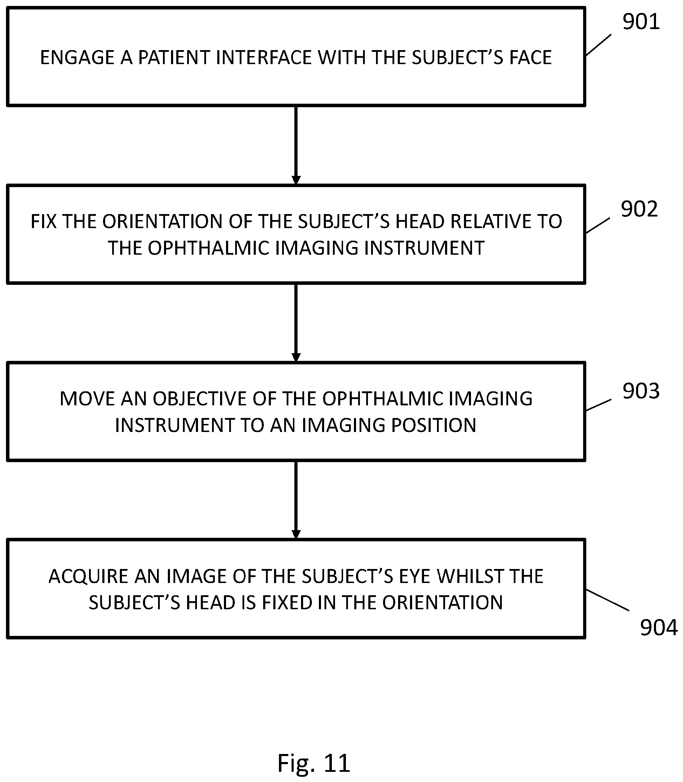

Turning now to Figure 11, a flow chart outlining a method of imaging an eye of a subject 32 with an ophthalmic imaging instrument 10 whilst fixing the orientation of the subject's head 36 relative to the ophthalmic imaging instrument 10 with a patient interface 12 is shown. In Step 901 the subject's face 34 engages the patient interface 12. Engaging the patient interface 12 with the subject's face 34 may comprise bringing the face 34 of the subject into contact with the patient interface 12. For example, the subject 32 may move toward the patient interface 12 from an upright seated position in which the subject leans towards the patient interface to bring the face 34 of the subject into contact with the patient interface 12.

Once the subject's face 34 has engaged the patient interface 12 the patient interface 12 fixes the orientation of the subject's head 36 relative to the ophthalmic imaging instrument 10 in Step 902. The patient interface 12 may fix the subject's head 36 in an orientation relative to the ophthalmic imaging instrument 10 by contacting a first or upper portion 34-1 of the face 34 overlying a frontal bone of the head 36 and respective second or left and right portions 34-2 of the face 34 overlying each zygomatic bone of the head 36 when the face 34 of the subject 32 is engaged with the patient interface 12. Contacting the frontal bone of the face 34 and each of the zygomatic bones with the patient interface 12 has the effect of anchoring the subject's head 36 relative to the ophthalmic imaging instrument 10 such that the subject 32 cannot change the orientation of their head 36 without first disengaging one or more of the: upper portion, left portion and right portion of the subject's face 34 from the patient interface 12.

In embodiments where the ophthalmic imaging instrument 10 comprises a moveable objective 15 the method may comprise Step 903 in which the objective 15 is moved from a retracted position to an imaging position along a movement axis 16. Moving the objective 15 from the retracted position to the imaging position may comprise moving the objective 15 along the movement axis 16 whilst the subject's head 36 is fixed in an orientation relative to the ophthalmic imaging instrument 10 by the patient interface 12. Moving the objective 15 may comprise moving the image acquisition apparatus 14.

In Step 904 an image of the subject's eye is acquired by the ophthalmic imaging instrument 10 whilst the face 34 of the subject 32 is engaged with the patient interface 12 and thus the orientation of the subject's head 36 is fixed relative to the ophthalmic imaging instrument 10.

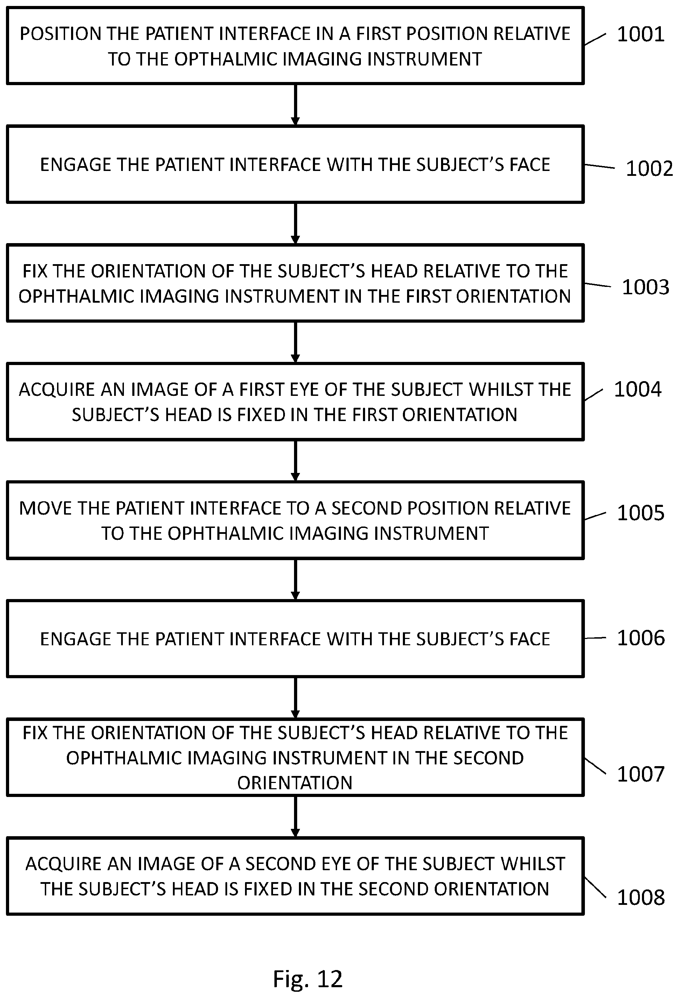

Figure 12 is a flow chart outlining a method of imaging both eyes of a subject 32 with an ophthalmic imaging instrument 10 whilst fixing the orientation of the subject's head 36 relative to the ophthalmic imaging instrument 10 with a patient interface 12. In Step 1001 the patient interface 12 is positioned in a first orientation relative to the ophthalmic imaging instrument 10. For example, the patient interface 12 may be positioned in the first position (left position) that is suitable for imaging the right eye of the subject 32. In Step 1002, when the patient interface 12 is in the first position the subject 32 engages the patient interface 12 with their face 34 as described above in relation to Step 901 of Figure 11.

Once the subject's face 34 engages the patient interface 12 the orientation of the subject's head 36 is fixed relative to the ophthalmic imaging instrument 10 in a first orientation in Step 1003. In the example when the first position of the patient interface 12 is suitable for imaging the right eye of the subject 32 and the subject's head 36 is fixed in an orientation in which the subject's head 36 is facing to the left of the movement axis 16 of the objective 15 (from the perspective of the subject). In Step 1004 an image of the first eye of the subject 32 is acquired whilst the orientation of the head 36 of the subject is fixed. In some embodiments the objective 15 may be moved from a retracted position to an imaging position prior to acquiring the image of the first eye. Furthermore, after the image of the first eye has been acquired the objective 15 may be moved from the imaging position to the retracted position.

After the image of the subject's first eye has been acquired the subject 32 may disengage the patient interface 12 and the patient interface 12 may be moved from the first position to the second position in Step 1005. Moving the patient interface 12 from the first position to the second position may comprise sliding the patient interface 12 along an arcuate path such that when the patient interface 12 is in the first position the patient interface 12 is orientated at a first angle relative to the movement axis 16 of the objective 15 and when the patient interface 12 is in the second position the patient interface 12 is orientated at a second angle relative to the movement axis 16 of the objective 15 wherein the first angle is different to the second angle.

In Step 1006 the subject 32 engages the patient interface 12 with the subject's face 34 whilst the patient interface 12 is in the second position. In Step 1007 the subject's head 32 is fixed in the second orientation relative to the ophthalmic imaging instrument 10 by the patient interface 12. The second orientation of the subject's head 36 may be a position that is suitable for imaging the left eye of the subject 32 and the subject's head 36 may be fixed in an orientation in which the subject's head 36 is facing to the right of the movement axis 16 of the ophthalmic imaging instrument 10 (from the perspective of the subject). In Step 1008 an image of the second eye of the subject 32 is acquired whilst the orientation of the head 36 of the subject 32 is fixed in the second orientation. Prior to acquiring the image of the second eye the objective of the ophthalmic imaging instrument 10 may be moved to the imaging position for imaging the second eye. For example, the objective 15 may be actuated from the retracted position to an imaging position.

Moving the objective 15 between the retracted position and imaging position may comprise acquiring images of an anterior region of the eye prior to and during movement of the objective 15. For example, a stereo ranging system (as described above) for measuring a distance between the objective 15 and the eye may be used to image an anterior region of the eye and to determine a distance between the objective and the anterior region of the eye. Moving the objective 15 from the retracted position to the imaging position may be based on the determined distance between the objective 15 and the eye.

Furthermore, images acquired by the stereo ranging system may be monitored for an obstruction that obscures the view of the anterior region of the eye. For example, as the objective 15 is actuated from the retracted position to the imaging position one or more anatomical features of the subject's face 34 such as the nose or brow may at least partially obscure the views of one or more of the cameras in the stereo ranging system. The method may comprise monitoring the images captured by the stereo ranging system for obstructions. If an obstruction is detected in the images the method may comprise one or more of: stopping movement of the objective, generating an alert to a clinician that the cameras have been obstructed and retracting the objective 15 until the obstruction has been removed. If an obstruction is detected the subject may disengage the patient interface 12 such that the orientation of the patient interface 12 relative to the ophthalmic imaging system 10 can be altered. Changing the orientation of the patient interface 12 beneficially allows the angle of the subject's head 36 to be varied relative to the objective 15 such that the position of anatomical features of the subject's face 34 can be controlled to prevent obscuration of images captured by the stereo ranging system.

Additionally or alternatively, the method may comprise monitoring a separation between the objective 15 and the face 34 of the subject 32 whilst moving the objective 15 from the retracted position to the imaging position and stopping moving the objective 15 from the retracted position to the imaging position if a collision is predicted. Predicting when a collision is about to occur may comprise measuring the distance between the objective 15 and the subject's face 34. This may be performed by proximity sensors mounted on the objective or by the stereo ranging system for example.

In the foregoing description, example aspects are described with reference to several example embodiments. Accordingly, the specification should be regarded as illustrative, rather than restrictive. Similarly, the figures illustrated in the drawings, which highlight the functionality and advantages of the example embodiments, are presented for example purposes only. The architecture of the example embodiments is sufficiently flexible and configurable, such that it may be utilized in ways other than those shown in the accompanying figures.

While various example embodiments of the present invention have been described above, it should be understood that they have been presented by way of example, and not limitation. It will be apparent to persons skilled in the relevant art(s) that various changes in form and detail can be made therein. Thus, the present invention should not be limited by any of the above-described example embodiments, but should be defined only in accordance with the following claims and their equivalents. It is to be understood that any procedures recited in the claims need not be performed in the order presented.

While this specification contains many specific embodiment details, these should not be construed as limitations on the scope of any inventions or of what may be claimed, but rather as descriptions of features specific to particular embodiments described herein. Certain features that are described in this specification in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination, and the claimed combination may be directed to a sub-combination or variation of a sub-combination.

## Claims

1. An ophthalmic imaging instrument (10) for imaging an eye (30) of a subject (32), comprising:
a patient interface (12) with which a face (34) of a head (36) of the subject (32) is to engage during the imaging of the eye (30), the patient interface (12) being arranged to fix an orientation of the head (36) relative to the ophthalmic imaging instrument (10) by contacting an upper portion (34-1) of the face (34) overlying a frontal bone of the head (36) and respective left and right portions (34-2) of the face (34) overlying each zygomatic bone of the head (36) when the face (34) of the subject (32) engages with the patient interface (12); and
an image acquisition apparatus (14) arranged to acquire an image of the eye (30) while the face (34) of the subject (32) is engaged with the patient interface (12).

2. The ophthalmic imaging instrument (10) according to Claim 1, wherein the patient interface (12) is arranged to avoid contacting a nose (38) of the subject (32) and a lower portion (34-3) of the face (34) overlying a maxilla of the head (36) when the face (34) of the subject (32) is engaged with the patient interface (12).

3. The ophthalmic imaging instrument (10) according to Claim 2, wherein the patient interface (12) has an elongate contact surface comprising a central portion (101) arranged to contact the first portion (34-1) of the face (34) when the face (34) of the subject (32) is engaged with the patient interface (12), the contact surface extending to each of a first end portion (102) of the contact surface and a second end portion (103) of the contact surface, the first end portion (102) and the second end portion (103) being arranged to respectively contact the left and right portions (34-2) of the face (34) when the face (34) of the subject (32) is engaged with the patient interface (12).

4. The ophthalmic imaging instrument (10) according to any preceding claim, wherein the patient interface (12) is orientated such that, when the subject (32) leans in from an upright seated position to engage with the patient interface (12), the head (36) of the subject (32) has a downward inclination which is greater than in the upright seated position.

5. The ophthalmic imaging instrument (10) according to any preceding claim, wherein
the image acquisition apparatus (14) comprises an objective (15) which is movable along a movement axis (16) towards and away from the eye (30) while the face (34) of the subject (32) is engaged with the patient interface (12), and
the patient interface (12) is arranged to fix the orientation of the head (36) relative to the movement axis (16) such that the objective (15) can be moved along the movement axis (16) to a position for acquiring the image of the eye (30).

6. The ophthalmic imaging instrument (10) according to Claim 5, further comprising:
a base (17), on which the image acquisition apparatus (14) is slidably mounted; and
a gantry (18) which extends from the base (17) and supports the patient interface (12),
wherein the image acquisition apparatus (14) is slidable relative to the base (17) such that that the objective (15) is moveable along the movement axis (16) towards and away from the eye (30) when the face (34) of the subject (32) is engaged with the patient interface (12).

7. The ophthalmic imaging instrument (10) according to Claim 5 or Claim 6, further comprising a stereo camera ranging system for measuring a distance between the objective (15) and the eye (30) when the face of the subject is engaged with the patient interface, the stereo camera ranging system comprising a first camera (19a) and a second camera (19b) arranged to image the eye (30) from different locations relative to the objective (15), wherein the patient interface (12) is arranged to fix the orientation of the head (36) relative to the ophthalmic imaging instrument (10) such that each of the first camera (19a) and the second camera (19b) has a respective view of the eye (30) which is unobstructed by a facial feature of the subject (32).

8. The ophthalmic imaging instrument (10) according to any preceding claim, wherein the patient interface (12) is operable to fix the orientation of the head (36) relative to the ophthalmic imaging instrument (10) in:
a first relative orientation for imaging a right eye of the subject (32) by the ophthalmic imaging instrument (10); and
a second relative orientation for imaging a left eye of the subject (32) by the ophthalmic imaging instrument (10), the second relative orientation being different to the first relative orientation.

9. The ophthalmic imaging instrument (10) according to Claim 8 when dependent on Claim 6, wherein the gantry (18) comprises a curved guide rail (18a), and the patient interface (12) is slidably mounted on the curved guide rail (18a) so as to be moveable between:
a first position on the guide rail, at which the patient interface (12) is arranged to fix the orientation of the head (36) relative to the ophthalmic imaging instrument (10) in the first relative orientation for imaging the right eye of the subject (32); and
a second position on the guide rail, at which the patient interface (12) is arranged to fix the orientation of the head (36) relative to the ophthalmic imaging instrument (10) in the second relative orientation for imaging the left eye of the subject (32).

10. A patient interface (12) attachable to an ophthalmic imaging instrument (10), wherein the patient interface (12), when attached to the ophthalmic imaging instrument (10) and engaged with a face (34) of a subject (32), is arranged to fix an orientation of a head (36) of the subject (32) relative to the ophthalmic imaging instrument (10) by contacting an upper portion (34-1) of the face (34) overlying a frontal bone of the head (36) and respective left and right portions (34-2) of the face (34) overlying each zygomatic bone of the head (36), such that the ophthalmic imaging instrument (10) can acquire an image of the eye (30).

11. The patient interface (12) according to Claim 10, wherein the patient interface (12) is arranged to avoid contacting a nose (38) of the subject (32) and a lower portion (34-3) of the face (34) overlying a maxilla of the head (36) when the face (34) of the subject (32) is engaged with the patient interface (12).

12. The patient interface (12) according to Claim 11, wherein the patient interface (12) has an elongate contact surface comprising a central portion (101) arranged to contact the upper portion (34-1) of the face (34) when the face (34) of the subject (32) is engaged with the patient interface (12), the contact surface extending to each of a first end portion (102) of the contact surface and a second end portion (103) of the contact surface, the first end portion (102) and the second end portion (103) being arranged to respectively contact the left and right portions (34-2) of the face (34) when the face (34) of the subject (32) is engaged with the patient interface (12).

13. The patient interface (12) according to Claim 12, wherein the elongate contact surface further comprises a first arcuate portion (105) and a second arcuate portion (106) that extend from opposing ends of the central portion (101), each of the first arcuate portion (105) and the second arcuate portion (106) extending from a respective end of the central portion (101) to a respective one of the first end portion (102) and the second end portion (103) along a respective arcuate path which is dimensioned to wrap around an eye socket of the subject (32) when the face (34) of the subject (32) is engaged with the patient interface (12).

14. The patient interface (12) according to Claim 13, wherein the first end portion (102) of the contact surface is provided at an end of the first arcuate portion (105) which is distal from the central portion (101), and the second end portion (103) of the contact surface is provided at an end of the second arcuate portion (106) which is distal from the central portion (101).

15. The patient interface (12) according to Claim 13 or Claim 14, wherein the first arcuate portion (105) and the second arcuate portion (106) are arranged such that a gap is provided between the first end portion (102) and the second end portion (103), and wherein when the face (34) of the subject (32) engages the patient interface (12), the nose (38) of the subject (32) can be accommodated within the gap without being contacted by the patient interface (12).
